# EUROPÄISCHE PATENTANMELDUNG

(11) **EP 0 684 475 A2**
(43) Veröffentlichungstag der Anmeldung: **29.11.1995**
(21) Anmeldenummer: 95107913.6
(22) Anmeldetag: 23.05.1995
(51) Int. Cl.: G01N 33/564, G01N 33/573

(54) **Verfahren zum Nachweis des Vorliegens eines Insulin-abhängigen Diabetes mellitus**

(30) Priorität: 25.05.1994 DE 4418223
(71) Anmelder: BOEHRINGER MANNHEIM GMBH, D-68305 Mannheim-Waldhof (DE)
(72) Erfinder: Donie, Frederic, Dr., D-82377 Penzberg (DE); Kientsch-Engel, Rosemarie, Dr., D-82340 Feldafing (DE); Wozny, Manfred, Dr., D-82362 Weilheim (DE)
(74) Vertreter: Böhm, Brigitte, Dipl.-Chem. Dr.

(57) **Zusammenfassung**

Zum Nachweis des Vorliegens eines Insulin-abhängigen Diabetes mellitus (IDDM) bringt man in einem Immunoassay eine Blutprobe eines Patienten in Kontakt mit einem Reagenz, welches Lactatdehydrogenase (LDH) oder mindestens eine ihrer Untereinheiten M oder H oder mindestens ein Fragment hiervon enthält, und weist die Bildung von Komplexen mit im Patientenblut enthaltenen Antikörpern nach an sich bekannten Methoden nach. Ein Reagenz zum Nachweis des Vorliegens eines Insulin-abhängigen Diabetes mellitus enthält LDH oder zumindest eine ihrer Untereinheiten M oder H oder mindestens ein Fragment hiervon. Ein diagnostischer Testkit zum Nachweis des Vorliegens eines Insulinabhängigen Diabetes mellitus enthält ein erfindungsgemäßes Reagenz sowie weitere Komponenten zur Druchführung eines Immunoassays.

## Beschreibung

Die vorliegende Erfindung betrifft ein Verfahren zum Nachweis des Vorliegens eines Insulin-abhängigen Diabetes mellitus (IDDM), Reagenzien, welche den Nachweis von IDDM erlauben, deren Verwendung sowie einen diagnostischen Testkit zum Nachweis des Vorliegens eines IDDM.

Insulin-abhängiger Diabetes mellitus (IDDM), auch Typ I-Diabetes genannt, ist verbunden mit dem Auftreten von Autoantikörpern gegen Proteine, insbesondere einem 64 kD Protein der Glutaminsäuredecarboxylase (GAD). Eine Übersicht über Autoantigene, deren Auftreten mit IDDM verbunden sind, findet sich in Harrison, Immunology Today, Vol. 13, Nr. 9 (1992), S. 348-352. Die Glutaminsäuredecarboxylase kann dabei offensichtlich in antigenen und nicht-antigenen Formen auftreten (Christie et al., Diabetologia (1992), 35: 380-384).

Aufgrund dieser Tatsache sind bereits Verfahren entwickelt worden, um das Vorliegen oder eine Prädisposition für IDDM nachzuweisen, welche auf dem Nachweis von Autoantikörper gegen GAD oder bestimmten anderen Antigenen der Inselzellen der Bauchspeicheldrüse, wie z.B. ICA 512 und ICA 12, beruhen (US-PS 5,200,318, WO92/05446, WO92/04632).

Es wurde jedoch festgestellt, daß mit den bekannten Methoden zum Nachweis von Autoantikörpern, welche bisher mit IDDM in Verbindung gebracht werden, nur eine höchstens ca. 80 %ige Sicherheit bei der Einordnung von Patientenseren bezüglich des Vorliegens eines IDDM bzw. einer Prädisposition hierfür erreicht werden kann.

Es bestand daher ein Bedürfnis, weitere Bestimmungverfahren zu entwickeln, welche eine höhere Nachweisgenauigkeit von IDDM-relevanten Autoantikörpern zeigen. Aufgabe der vorliegenden Erfindung war es, ein derartiges Nachweisverfahren zu entwickeln.

Gelöst wird diese Aufgabe erfindungsgemäß durch ein Verfahren zum Nachweis des Vorliegens eines Insulin-abhängigen Diabetes mellitus, bei dem man in einem Immunoassay eine Blutprobe eines Patienten in Kontakt bringt mit einem Reagenz, welches Lactatdehydrogenase (LDH) oder mindestens eine ihrer Untereinheiten M oder H oder mindestens ein Fragment hiervon enthält, und die Bildung von Komplexen mit im Patientenblut enthaltenen Autoantikörpern nach an sich bekannten Methoden nachweist.

Überraschenderweise ist im Rahmen der vorliegenden Erfindung festgestellt worden, daß Reagenzien enthaltend LDH in einem Immunoassay eine sehr genaue Aussage über das Vorliegen eines IDDM erlauben. Auch Prädispositionen für IDDM werden hiervon erfaßt.

Es ist hierbei nicht unbedingt nötig, daß das gesamte LDH-Molekül in dem Reagenz vorhanden ist, auch eine seiner Untereinheiten und insbesondere die M-Untereinheit bzw. ein Fragment hiervon kann ausreichend sein, wenn sie in einem Immunoassay Verwendung finden. Auch verschiedene Kombinationen der Untereinheiten der LDH sind im Rahmen der vorliegenden Erfindung verwendbar. Im erfindungsgemäßen Verfahren werden dann bevorzugt Komplexe aus LDH oder einer der eingesetzten LDH-Untereinheiten und Anti-LDH-Antikörpern nachgewiesen. Schließlich ist es ebenfalls möglich, Oligomere von LDH oder LDH-Untereinheiten oder LDH-Fragmenten zu vermeiden.

Besonders bevorzugt ist es im Rahmen der vorliegenden Erfindung, ein Reagenz im Immunoassay einzusetzen, welches neben LDH oder mindestens einer ihrer Untereinheiten noch GAD oder GAD-Fragmente enthält sowie gegebenenfalls eines oder mehrere weitere Proteine, an die Diabetes-relevante Autoantikörper binden können. Im Immunoassay werden dann vorzugsweise alle gebildeten Komplexe aus im Reagenz enthaltenen Proteinen und im Patientenblut enthaltenen Autoantikörpern erfaßt. Es ist aber nicht unbedingt notwendig, daß für jedes der verwendeten Proteine tatsächlich Autoantikörper vorhanden sind. Vielmehr ist es auch möglich, daß das Vorhandensein eines der Proteine die Bindefähigkeit eines der anderen in einer Weise moduliert, daß dagegen gerichtete Autoantikörper im Immunoassay besser detektierbar werden. Es ist hierbei z.B. an eine Modulatorwirkung zu denken, bei der sich zwei oder mehr Proteine in einer solchen Weise beeinflussen, daß die Konformation mindestens eines Proteins so verändert wird, daß die Epitope für vorhandene IDDM-relevante Autoantikörper so exponiert werden, daß auch tatsächlich eine Immunreaktion stattfinden kann.

Das erfindungsgemäße Reagenz kann neben LDH-Homo-Oligomeren auch Hetero-Oligomere aus LDH, LDH-Untereinheit(en), LDH-Fragment(en) und anderen Proteinen, die an Diabetes-relevante Antikörper binden, insbesondere GAD oder Fragmente davon, enthalten.

Diese Oligomere/Aggregate können synthetisch hergestelt werden (z.B. kovalent, durch dem Fachmann bekannte proteinchemische Methoden) oder natürlich vorkommen (z.B. nicht kovalent verbundene Protein-Komplexe). Erfindungsgemäß zur Anwendung kommende Protein-Fragmente können durch Synthese aus Aminosäuren oder durch chemische/enzymatische Spaltung der Proteine hergestellt werden oder natürlich vorkommen.

Im erfindungsgemäßen Verfahren ist es möglich, sowohl isolierte native als auch rekombinant hergestellte LDH zu verwenden. Gleiches gilt für GAD oder andere Proteine.

Besonders bevorzugt wird als Immunoassay im Rahmen der vorliegenden Erfindung ein ELISA (Enzyme Linked Immuno Sorbent Assay) oder ein HIA (Homogenous Immuno Assay) durchgeführt. Prinzipiell sind jedoch auch alle anderen Arten von Immunoassays geeignet, so lange sie den Nachweis Diabetes-relevanter Autoantikörper ermöglichen.

Besonders bevorzugt koppelt man LDH oder mindestens eine ihrer Untereinheiten oder Fragmente an eine Festphase (ELISA) oder an Partikel, wie insbesondere Latexpartikel (HIA).

Diese Kopplung kann nach an sich bekannten Methoden erfolgen, wobei jedoch darauf geachtet werden muß, daß keine Konformationsänderung der Proteine in solcher Weise eintritt, daß die Kopplung der Autoantikörper unterbunden wird. In einer besonders bevorzugten Ausführungsform der Erfindung erfolgt die Kopplung über ein Bindepaar, wobei einer der Partner des Bindepaares an die Festphase oder an Partikel gebunden vorliegt und der andere Partner des Bindepaares an LDH, mindestens eine LDH-Untereinheit oder mindestens ein Fragment davon bzw. an GAD, GAD-Fragmente oder andere Diabetes-relevante Proteine gekoppelt vorliegt. Ein besonders bevorzugtes Bindepaar ist im Rahmen der Erfindung Streptavidin/Biotin, wobei es wiederum besonders bevorzugt ist, wenn die Festphase bzw. die Partikel mit Streptavidin konjugiert sind und LDH, LDH-Untereinheit(en), Fragment(e), GAD, GAD-Fragment(e) oder andere Proteine an Biotin gekoppelt sind.

Besonders bevorzugt wird der Nachweis des Komplexes aus Protein und Diabetes-relevanten Autoantikörper der Probe mit Hilfe von human Ig-bindenden Substanzen, wie Anti-human Ig-Antikörpern, Anti-human Leichtketten-Antikörper, Protein A, Protein G oder anderen dem Fachmann bekannten Mitteln durchgeführt, wobei insbesondere Anti-human IgG-Antikörper, Anti-human IgM-Antikörper oder Anti-human IgA-Antikörper zum Einsatz kommen können.

Besonders bevorzugt verwendet man markierte Anti-human Ig-Antikörper im erfindungsgemäßen Immunoassay.

Ein erfindungsgemäßer Immunoassay kann vorzugsweise auch so modifiziert werden, daß als Bestätigungstest zusätzlich underivatisierte LDH oder mindestens eine ihrer Untereinheiten zugesetzt wird und man lediglich dann, wenn diese zusätzliche underivatisierte LDH, LDH-Untereinheit oder/und LDH-Fragment das Markierungssignal im Vergleich zum Signal ohne underivatisierte LDH bzw. LDH-Untereinheit oder Fragment beeinflußt, den Test als positiv wertet. Hierbei ist es wiederum bevorzugt, auch zusätzlich noch underivatisierte GAD oder GAD-Fragmente zuzusetzen.

Als Markierungen der human Ig-bindenden Substanzen sind alle in Immunoassays einsetzbaren Markierungssysteme geeignet. Vorzugsweise wird mit nicht-radioaktiven Markierungen gearbeitet und von den gängigen Markierungen seien hier lediglich Enzymmarkierung und Fluoreszenzmarkierung genannt. Andere Markierungsmethoden sind dem Fachmann bekannt und leicht auf ihre Tauglichkeit im erfindungsgemäßen Immunoassay zu untersuchen.

Ein weiterer Gegenstand der vorliegenden Erfindung ist ein Reagenz zum Nachweis des Vorliegens eines IDDM, welches LDH oder zumindest eine ihrer Untereinheiten M oder H bzw. mindestens ein Fragment davon enthält. Wie bereits oben zum erfindungsgemäßen Verfahren ausgeführt, kann auch eine Kombination der Untereinheiten M oder/und H oder von Fragmenten davon im erfindungsgemäßen Reagenz vorhanden sein.

Das erfindungsgemäße Reagenz dient insbesondere zum Nachweis von IDDM mit Hilfe des erfindungsgemäßen Verfahrens. Es kann isolierte native oder rekombinante LDH oder deren Untereinheit(en), Fragment(e) enthalten. Besonders bevorzugt enthält das erfindungsgemäße Reagenz noch weitere Proteine oder Fragmente davon, an die Diabetes-relevante Autoantikörper binden können. Vorzugsweise enthält es zusätzlich noch GAD oder GAD-Fragmente.

Es ist im Rahmen der vorliegenden Erfindung bevorzugt, daß im Reagenz LDH, LDH-Untereinheit, LDH-Fragment(e) und gegebenenfalls andere Proteine, an die Diabetes-relevante Autoantikörper binden, insbesondere GAD oder GAD-Fragmente, in einer Form vorliegen, die den Einsatz in einem Immunoassay ermöglicht. Hierbei ist insbesondere zu denken an die Möglichkeit einer Kopplung an eine Festphase und zwar insbesondere über ein Bindungspaar, wobei LDH, LDH-Untereinheit, LDH-Fragment und gegebenenfalls andere Proteine, wie inbesondere GAD oder GAD-Fragmente, an einen Partner eines Bindesystems gekoppelt vorliegt, und zwar vorzugsweise an Biotin gekoppelt. Bei Verwendung einer Festphase oder Partikeln, welche mit Streptavidin beschichtet oder anders konjugiert ist, kann dann leicht eine Bindung an die Festphase erfolgen.

In einer bevorzugten Ausführungsform des erfindungsgemäßen Reagenzes, welche insbesondere für die Durchführung eines Bestätigungstests für obigen Immunoassays geeignet ist, enthält das Reagenz noch underivatisierte LDH oder/und LDH-Untereinheit oder/und LDH-Fragment und gegebenenfalls noch weitere underivatisierte Proteine oder Fragmente davon, an die Diabetesrelevante Autoantikörper binden. Dies ist besonders bevorzugt wiederum GAD oder GAD-Fragmente.

Ein weiterer Gegenstand der vorliegenden Erfindung ist ein Reagenz zum Nachweis des Vorliegens eines Insulin-abhängigen Diabetes mellitus, erhältlich durch Isolierung der Proteinfraktion aus LDH-haltigen Geweben und anschließender chromatographischer Auftrennung, wobei man bei der Elution diejenigen Fraktionen sammelt, welche LDH-Aktivität (enzymatische Aktivität oder immunologische Aktivität) zeigen.

Ein wiederum weiterer Gegenstand der vorliegenden Erfindung ist ein Reagenz zum Nachweis des Vorliegens eines Insulin-abhängigen Diabetes mellitus, erhältlich durch Isolierung der Proteinfraktion aus GAD-haltigen Geweben und anschließender chromatographischer Auftrennung, wobei man bei der Elution diejenigen Fraktionen sammelt, welche LDH-Aktivität (enzymatische Aktivität oder immunologische Aktivität) zeigen.

Wiederum ein weiterer Gegenstand der vorliegenden Erfindung ist ein Reagenz zum Nachweis des Vorliegens eines Insulin-abhängigen Diabetes mellitus, erhältlich durch Isolierung der Proteinfraktion aus sowohl GAD- als auch LDH-haltigen Geweben und anschließender chromatographischer Auftrennung, wobei man bei der Elution diejenigen Fraktionen sammelt, welche GAD- oder/und LDH-Aktivität (enzymatische Aktivität oder immunologische Aktivität) zeigen.

Es ist dabei bevorzugt, ein Detergenz, insbesondere ein zwitterionisches Detergenz, wie z.B. CHAPSO, zu verwenden.

Mit den erfindungsgemäßen Reagenzien wird es ermöglicht, Immunoassays durchzuführen, welche in besonders hohem Maße IDDM-relevante Autoantikörper auffinden, weshalb die Aussagekraft dieser Immunoassays besonders hoch ist.

Das erfindungsgemäße Reagenz enthält bevorzugt noch Substanzen, welche das Reagenz stabilisieren und damit eine längere Lagerzeit ermöglichen. Solche Substanzen können beispielsweise Pyridoxalphosphat, Zusatzstoffe wie Reduktionsmittel, insbesondere DTT (Dithiothreitol) oder Zucker, wie insbesondere Saccharose sein.

Ein wiederum weiterer Gegenstand der vorliegenden Erfindung ist ein diagnostischer Testkit. Ein derartiger diagnostischer Testkit enthält ein erfindungsgemäßes Reagenz zum Nachweis von IDDM sowie weitere Komponenten zur Durchführung eines Immunoassays. Hierbei ist zu denken an Festphasen, die die Durchführung eines ELISA ermöglichen, Partikel, welche die Durchführung eines HIA ermöglichen, Puffersubstanzen, Komponenten zum Nachweis von Markierungen und andere, dem Fachmann gut bekannte Merkmale.

In einer bevorzugten Ausführungsform enthält der erfindungsgemäße diagnostische Testkit eine mit Streptavidin beschichtete Festphase bzw. Streptavidin-beschichtete Partikel.

Desweiteren enthält der diagnostische Testkit human Ig-bindende Substanzen, wie Anti-human Ig-Antikörper, Anti-human Leichtketten-Antikörper, Protein A, Protein G oder andere dem Fachmann bekannte Mittel, welche den Nachweis der Komplexbildung zwischen einem der Proteine, an die Diabetes-relevante Autoantikörper binden und den Autoantikörpern ermöglichen. Bevorzugt sind dies im Rahmen der vorliegenden Erfindung Anti-human IgG-Antikörper, Anti-human IgM-Antikörper oder Anti-human-IgA-Antikörper.

Diese Antikörper sind in einer bevorzugten Ausführungsform der Erfindung mit einer Markierung versehen, insbesondere einer Markierung, welche einen leichten und automatisierbaren Nachweis erlaubt.

Ein weiterer Gegenstand der vorliegenden Erfindung ist die Verwendung von LDH, mindestens einer LDH-Untereinheit oder mindestens eines Fragmentes davon zum Nachweis des Vorliegens eines Insulin-abhängigen Diabetes mellitus.

Im Rahmen der vorliegenden Erfindung ist es auch möglich, Oligomere von LDH, mindestens einer LDH-Untereinheit oder mindestens eines Fragmentes hiervon und gegebenenfalls weiteren Proteinen oder Fragmenten davon, an welche IDDM-relevante Antiautokörper binden, und insbesondere GAD oder GAD-Fragmenten zu verwenden. Es ist dabei sowohl möglich, Homo-Oligomere aus LDH, LDH-Fragmenten oder Untereinheiten zu bilden, oder aber Hetero-Oligomere aus LDH mit einem anderen Protein.

Zusammenfassend kann festgestellt werden, daß die vorliegende Erfindung ein Verfahren bereitstellt, das den Nachweis des Vorliegens eines IDDM erlaubt. Erfindungsgemäß werden desweiteren die notwendigen Reagenzien sowie ein Testkit bereitgestellt, mit dem der erfindungsgemäße Nachweis geführt werden kann. Das erfindungsgemäße Verfahren erlaubt eine besonders hohe Aussagekraft über das Vorliegen des IDDM bzw. einer Prädisposition hierfür. LDH kann dabei nicht nur in den beschriebenen Immunoassays, sondern auch in Verfahren wie Immunpräzipitation, Immunotrapping (LDH-Aktivitätshemmung), Nachweis reaktiver T-Zellen und anderen dem Fachmann bekannten Verfahren zur Diagnose von IDDM verwendet werden.

Die vorliegende Erfindung wird durch die folgenden Beispiele weiter erläutert.

### BEISPIEL 1

Herstellung einer Antigenpräparation, die sowohl GAD und LDH als auch Fragmente hiervon in monomerer oder oligomerer Form enthält
Herstellungsprinzip: Schweine-Cerebellum wird homogenisiert und nach einem Hitze-/Säureschritt chromatographisch über DEAE-Sepharose, Hydroxylapatit-Ultrogel, Q-Sepharose und Superdex 200 aufgereinigt.

### Puffer:

- A:: 0,005 M KPᵢ pH 7,1, 2,5 mM EDTA, 1 % PEG 300, 2 mM DTT, 0,2 mM PLP und Proteaseinhibitoren
- B:: 0,005 M KPᵢ pH 7,1, 1 mM DTT, 0,02 mM PLP
- C:: 0,005 M KPᵢ pH 7,1, 0,3 M NaCl, 1 mM DTT, 0,02 mM PLP
- D:: 0,3 M KPᵢ pH 7,1, 1 mM DTT, 0,02 mM PLP
- E:: 0,005 M KPᵢ pH 7,1, 1 mM DTT, 0,02 mM PLP, 0,2 % ChAPSO
- F:: 0,2 M KPᵢ pH 7,1, 1 mM DTT, 0,02 mM PLP, 0,2 % ChAPSO
- G:: 0,01 M KPᵢ pH 7,1, 0,1 M NaCl, 1 mM DTT, 0,02 mM PLP, 0,2 % ChAPSO
- H:: 0,02 M KPᵢ pH 7,1, 0,05 M NaCl, 1 mM DTT, 0,02 mM PLP

Alle nachfolgenden Schritte werden bei 4°C durchgeführt, wenn nichts anderes vermerkt ist.

Schweinecerebellum wird mit Puffer A homogenisiert. Anschließend wird das Homogenat auf pH 5,5 gebracht und auf ca. 45°C erhitzt. Nach 5 bis 10 min bei dieser Temperatur wird abgekühlt, neutralisiert und zentrifugiert.

Eine Chromatographiesäule mit DEAE-Sepharose (Pharmacia) wird mit Puffer B äquilibriert und der obige Überstand der Zentrifugation auf die Säule aufgezogen. Die Elution erfolgt mit einem Gradienten aus Puffer B und C. Die Fraktionen mit GAD-Aktivität werden weiterverarbeitet.

Eine Chromatographiesäule mit Hydroxylapatit-Ultrogel (IBF) wird mit Puffer B äquilibriert und die obigen gepoolten Fraktionen werden auf die Säule aufgezogen. Die Elution erfolgt mit einem Gradient aus Puffer B und D. Die Fraktionen mit GAD-Aktivität werden dialysiert gegen Puffer E ohne CHAPSO.

Das Dialysat wird mit CHAPSO versetzt (Endkonzentration 0,2 %) und auf eine Chromatographiesäule mit Q-Sepharose ff (Pharmacia) aufgezogen. Die Elution erfolgt mit einem Gradient aus Puffer E und F. Die Fraktionen, die möglichst viel LDH-Aktivität haben und vor der maximalen GAD-Aktivität eluieren, werden weiterverarbeitet.

Eine Chromatographiesäule mit Superdex 200 (Pharmacia) wird mit Puffer G äquilibriert und obige gepoolten Fraktionen werden hieran gelfiltriert. Die Fraktionen mit GAD-Aktivität werden als Antigenpräparation verwendet.

### Verwendete Abkürzungen:

- CHAPSO: 3-[(3-Cholamidopropyl)dimethylammonio]-2-hydroxy-1-propansulfonat
- DTT: Dithiothreitol
- PLP: Pyridoxalphosphat
- PEG: Polyethylenglycol

### BEISPIEL 2

### Durchführung eines Antikörpertests

In jede Kavität einer mit ThermoRSA-Streptavidin vorbeschichteten Mikrotiterplatte werden 100 µl biotinylierte Antigenpräparation (GAD und LDH enthaltend), isoliert aus Schweinegehirn, in einer für die jeweilige Charge optimalen Konzentration (1 bis 3 µg/ml in Inkubationspuffer aus dem Boehringer Mannheim Enzymun-Test® Anti-HIV 1+2, Best.Nr. 1144561) pipettiert und 30 min bei Raumtemperatur inkubiert. Danach wird die Platte dreimal gewaschen mit jeweils 350 µl 50 mmol/l Kaliumphosphat, pH 7,0 mit Zusatz von 0,1 % (W/v) CHAPSO (3-[(3-Cholamidopropyl)dimethylammonio]-2-hydroxy-1-propansulfonat). Humanserum wird 1+25 verdünnt in Inkubationspuffer aus dem Boehringer Mannheim Enzymun-Test® Anti-HIV 1+2. Diese so verdünnten Proben werden in einem Volumen von 100 µl/Kavität bei Raumtemperatur mit Schütteln der Mikrotiterplatte 1 Stunde inkubiert. Die Proben werden anschließend abgesaugt und die Platten 3x gewaschen wie oben. Der POD-gekoppelte Nachweisantikörper aus Schaf mit Bindungsspezifität für Human-IgG wird in Konjugatpuffer aus dem Boehringer Mannheim Enzymun-Test® Anti-HIV 1+2 auf eine Konzentration von 75 mU/ml verdünnt und in jeder Kavität 100 µl dieser verdünnten Lösung 1 Stunde bei Raumtemperatur unter Schütteln inkubiert. Die Flüssigkeit wird abgesaugt und die Platte wieder 3x gewaschen wie oben. Der Farbstoff ABTS® wird zu einer Konzentration von 1 mg/ml in Enzymun-Test® Substratpuffer gelöst und 100 µl/Kavität bei Raumtemperatur ohne Schütteln inkubiert. Nach ca. 30 min wird die Extinktion in einem Mikrotiter-Plattenphotometer abgelesen. Meßwellenlänge ist 405 nm, Referenzwellenlänge 492 nm. Der Leerwert sind zwei unbehandelte Kavitäten, die nur Substrat-/Farbstofflösung enthalten. Der Mittelwert der Extinktion der beiden Leerwert-Kavitäten wird von allen anderen Extinktionen abgezogen.

## Patentansprüche

1. Verfahren zum Nachweis des Vorliegens eines Insulinabhängigen Diabetes mellitus (IDDM),
**dadurch gekennzeichnet**
daß man in einem Immunoassay eine Blutprobe eines Patienten in Kontakt bringt mit einem Reagenz, welches Lactatdehydrogenase (LDH) oder mindestens eine ihrer Untereinheiten M oder H oder mindestens ein Fragment hiervon enthält, und die Bildung von Komplexen mit im Patientenblut enthaltenen Antikörpern nach an sich bekannten Methoden nachweist.

2. Verfahren nach Anspruch 1,
**dadurch gekennzeichnet,**
daß man im Immunoassay ein Reagenz verwendet, welches neben LDH oder mindestens einer ihrer Untereinheiten oder mindestens eines Fragments hiervon noch Glutaminsäuredecarboxylase (GAD) oder GAD-Fragmente oder mindestens ein anderes Protein enthält, an das Diabetes-relevante Antikörper binden können und die Bildung aller Komplexe im Immunoassay erfaßt.

3. Verfahren nach einem der vorhergehenden Ansprüche,
**dadurch gekennzeichnet,**
daß man LDH oder mindestens eine ihrer Untereinheiten oder mindestens ein Fragment hiervon an eine Festphase oder Partikel koppelt.

4. Verfahren nach Anspruch 3,
**dadurch gekennzeichnet,**
daß die Kopplung über ein Bindepaar und insbesondere über das Bindepaar Streptavidin/Biotin erfolgt, wobei einer der Partner des Bindepaares an eine Festphase oder Partikel gebunden vorliegt und der andere Partner des Bindepaars an LDH oder mindestens eine LDH-Untereinheit oder mindestens ein Fragment hiervon gekoppelt vorliegt.

5. Verfahren nach einem der Anprüche 3 und 4,
**dadurch gekennzeichnet,**
daß man einen Bestätigungstest durchführt, bei dem man zusätzlich underivatisierte LDH oder mindestens eine ihrer Untereinheiten oder mindestens ein Fragment hiervon oder Oligomere davon zusetzt oder daß man zusätzlich zur LDH underivatisierte GAD, GAD-Fragmente oder Oligomere davon zusetzt und lediglich dann, wenn durch die zusätzliche underivatisierte LDH das Markierungssignal, im Vergleich zum Signal ohne underivatisierte LDH, beieinflußt wird, den Test als positiv wertet.

6. Reagenz zum Nachweis des Vorliegens eines Insulinabhängigen Diabetes mellitus,
**dadurch gekennzeichnet,**
daß es LDH oder zumindest eine ihrer Untereinheiten M oder H oder mindestens ein Fragment oder Oligomere hiervon enthält.

7. Reagenz nach Anspruch 6,
**dadurch gekennzeichnet**,
daß es zusätzlich GAD, GAD-Fragmente oder andere Proteine oder Oligomere hiervon enthält, an die Diabetes-relevante Autoantikörper binden können.

8. Reagenz nach Anspruch 6 oder 7,
**dadurch gekennzeichnet,**
daß LDH, LDH-Untereinheit, LDH-Fragment und gegebenenfalls andere Proteine, an die Diabetes-relevante Autoantikörper binden, vorzugsweise GAD oder GAD-Fragmente, an einen Partner eines Bindepaares gekoppelt vorliegt, vorzugsweise an Biotin gekoppelt.

9. Reagenz zum Nachweis des Vorliegens eines Insulinabhängigen Diabetes mellitus, erhältlich durch Isolierung einer Proteinfraktion aus LDH- und/oder GAD-haltigen Geweben und anschließender chromatographischer Auftrennung, wobei man bei der Elution diejenigen Fraktionen sammelt, welche LDH-Aktivität (enzymatische Aktivität oder immunologische Aktivität) zeigen.

10. Reagenz zum Nachweis des Vorliegens eines Insulinabhängigen Diabetes mellitus, erhältlich durch Isolierung einer Proteinfraktion aus sowohl GAD- als auch LDH-haltigen Geweben und anschließender chromatographischer Auftrennung, wobei man bei der Elution diejenigen Fraktionen sammelt, welche GAD- oder/und LDH-Aktivität (enzymatische Aktivität oder immunologische Aktivität) zeigen.

11. Diagnostischer Testkit zum Nachweis des Vorliegens eines Insulin-abhängigen Diabets mellitus,
**dadurch gekennzeichnet,**
daß er ein Reagenz nach einem der Ansprüche 6 bis 10 enthält sowie weitere Komponenten zur Durchführung eines Immunoassays.

12. Diagnostischer Testkit nach Anspruch 11,
**dadurch gekennzeichnet,**
daß er eine mit Streptavidin beschichtete Festphase oder Partikel enthält.

13. Verwendung von LDH oder mindestens einer ihrer Untereinheiten oder mindestens eines Fragments oder Oligomers hiervon zum Nachweis des Vorliegens eines Insulin-abhängigen Diabetes mellitus.
